(19) Europäisches Patentamt
European Patent Office
Office européen des brevets

(11) Publication number: **0 304 965 A2**

(12) # EUROPEAN PATENT APPLICATION

(21) Application number: 88200323.9

(51) Int. Cl.4: C07C 59/68 , A01N 43/40

(22) Date of filing: 07.11.84

(30) Priority: 10.11.83 US 550328

(43) Date of publication of application: 01.03.89 Bulletin 89/09

(60) Publication number of the earlier application in accordance with Art.76 EPC: 0 142 328

(84) Designated Contracting States: AT BE CH DE FR GB IT LI NL SE

(71) Applicant: THE DOW CHEMICAL COMPANY
2030 Dow Center Abbott Road P.O. Box 1967
Midland Michigan 48640-1967(US)

(72) Inventor: Rogers, Richard B.
1837 Polk Street
Concord California 94521(US)

(74) Representative: Allard, Susan Joyce et al
BOULT, WADE & TENNANT 27 Furnival Street
London EC4A 1PQ(GB)

(54) **Fluorophenoxy compounds, herbicidal compounds and processes.**

(57) Certain novel fluorophenoxy derivatives, in particular phenoxyfluorophenoxyaklanoic acids and derivatives thereof are described. These novel compounds bear 1 and 2 fluorine substituents on the phenyl group which is attached to the alkanoic acid group. These novel compounds exhibit surprising preemergent and postemergent activity when used according to the method of the invention in the control of grassy weeds.

EP 0 304 965 A2

## FLUOROPHENOXY COMPOUNDS, HERBICIDAL COMPOSITIONS AND METHODS

This invention relates (a) to novel fluorophenoxy compounds, (b) to herbicidal compositions of such novel compounds and (c) to methods of using such compounds for the preemergent and postemergent control of grassy weeds in non-crop areas as well as in the presence of certain valuable crops such as soybeans, cotton and wheat.

Belgian Patent No. 834,495, issued February 2, 1976, as well as the published German patent application equivalent thereto, viz., No. 2,546,251, published April 29, 1976, describe 2-((4-pyridinyl-2-oxy)-phenoxy)-alkanoic acids, salts and esters having halo substitution in the 3- and/or 5-ring positions in the pyridine ring. Later references, e.g. published British Patent application 2,026,865 disclose such compounds having trifluoromethyl substitution on the pyridine ring and European Patent 0002800 describes the enhanced effect of the D-stereoisomers of such compounds.

The present invention is directed to novel fluorophenoxy compounds having herbicidal activity and which correspond to the formula

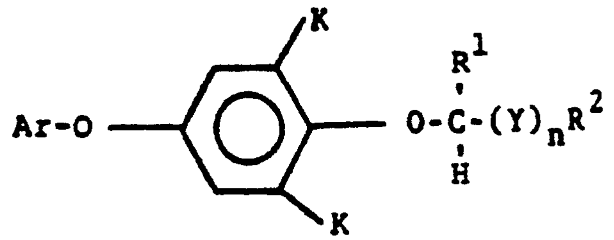

wherein Ar is a substituted or unsubstituted phenyl group;
K is H or F with the proviso that at least one K is F;
Y is a saturated or unsaturated alkyl groups containing an even number of carbon atoms;
N is 0 or 1;
$R^1$ is H or a $C_1$-$C_3$ alkyl group;
$R^2$ is a carboxyl group, an alkali metal, alkaline earth metal, ammonium or organic amine salt thereof; or an organic group containing N,O or S atoms that can be hydrolyzed and/or oxidised in plants or the soil to a carboxyl group that is in undissociated and/or dissociated form.

the invention is also directed to the novel stereoisomers of such compounds, the R-isomers having exceptional activity.

A variety of herbicidal compounds containing substituted phenoxy moieties joined via a bivalent -O- or -S- are described in the art. For example, U.S. Patent Nos. 4,046,553; 4,317,913; 4,267,336; 4,213,774; 4,324,627 and 4,309,547; U.S. Patent Application Serial Nos. 262,063 and 261,109, both filed July 30, 1980; European Patent 483 and European Patent Applications 1473; 4433; 50,019; 50,097; 75,840 and 83,556 all describe such compounds, methods of making them, compositions containing them and methods of utilizing said compositions. In general, the moieties bonded to the pendant -O- group of the phenoxy in the herbicidal compounds described in these references will also be suitable as the monovalent organic radicals represented by Ar and $R^2$ in the formula for the aforementioned novel compounds and, given the appropriate starting materials, the compounds of this invention can be prepared by methods described in the above-mentioned prior art, and can be utilized in compositions as described in said prior art.

Ar moieties include, but are not limited to,

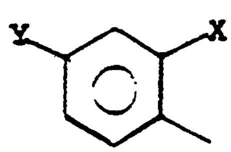

wherein X is hydrogen or halogen and Y is halogen, $CF_3$, $CHF_2$ or $CClF_2$.

In the group of the formula

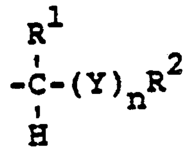

the group Y preferably contains from 2 to 18 carbon atoms, and $R^2$ is preferably selected from moieties corresponding to one of the following formulae:

-CN,

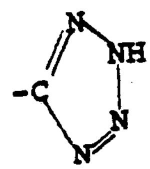

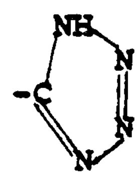

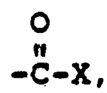

wherein X is halogen, or CN,

$-\overset{O}{\overset{\|}{C}}-O^{-}M^{+}$, wherein M is a metallic cation, ammonium or an organic amine cation typically, but not exclusively, containing alkyl (saturated or unsaturated), alicyclic, heterocyclic or aromatic groups, all unsubstituted or substituted with various other groups not limited to, but including, halo, cyano, nitro, and unsubstituted or substituted thiol, hydroxy, amino or carboxyl groups and, additionally, alicyclic, heterocyclic and aromatic groups substituted with unsubstituted or substituted saturated or unsaturated alkyl groups, for example, trifluoromethyl, chloromethyl, cyanomethyl and vinyl,

$$-CH_2OR^3,$$

$$-\overset{O}{\overset{\parallel}{C}}-R^3,$$

$$-CH_2O-\overset{O}{\overset{\parallel}{C}}-R^6,$$

$$-\overset{O}{\overset{\parallel}{C}}-OR^3,$$

$$-\overset{O}{\overset{\parallel}{C}}-SR^3,$$

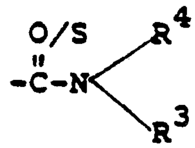
,

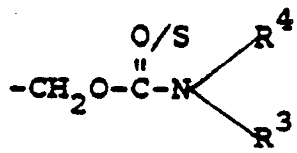
,

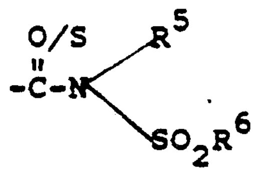
,

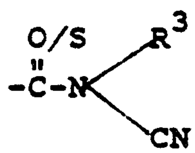
,

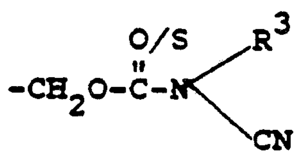
,

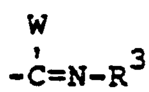
,

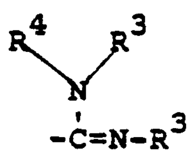
,

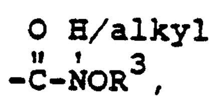
,

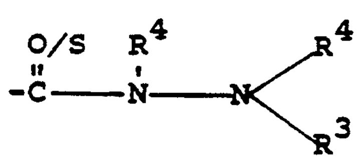
,

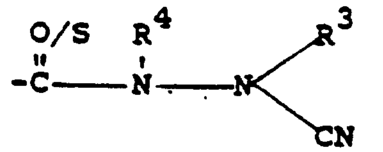 ,

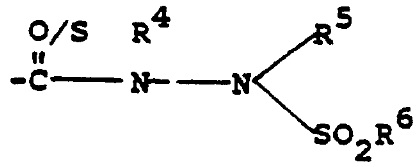 ,

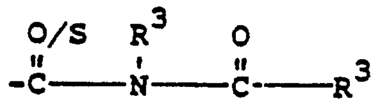 ,

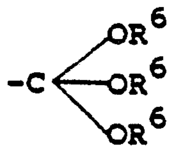 ,

$-C(SR^6)_3$ ,

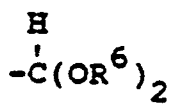 ,

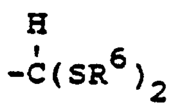 ,

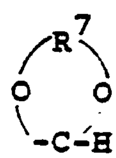 ,

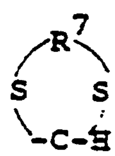 ,

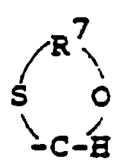 ,

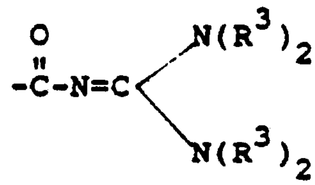
,

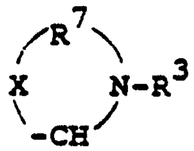 where X is S or O,

where W is halogen, alkoxy or alkylthio; $R^3$ is H or $R^6$; $R^4$ is H, alkoxy or $R^6$; $R^5$ is H, a metallic cation or $R^6$; and $R^6$ is an alkyl (saturated or unsaturated), alicyclic, heterocyclic or aromatic group, unsubstituted or substituted with various other groups not limited to, but including, halo, cyano, nitro and unsubstituted or substituted thiol, hydroxy, amino or carboxyl groups and, additionally, alicyclic, heterocyclic and aromatic groups substituted with unsubstituted or substituted saturated or unsaturated alkyl groups, for example, trifluoromethyl, chloromethyl, cyanomethyl and vinyl,

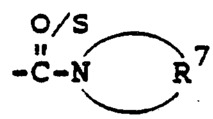
,

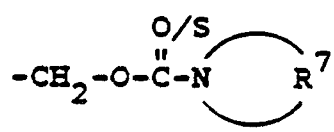
,

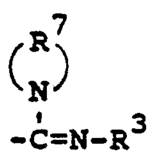
,

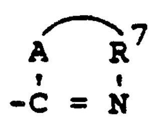 where A is O, S or N, or

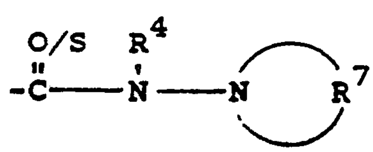
,

where $R^7$ completes an unsubstituted or substituted saturated heterocyclic ring system.

The above derivatives can be made by processes generally known to those skilled in the art and as described in the above-mentioned patents. For example, the corresponding acid chlorides can be reacted with a Grignard reagent to make the desired ketone derivative. Similarly, reaction of an acid chloride with KSH will provide the desired thiol acid. Thioamides may be prepared from the corresponding amide by reaction with $P_2S_5$ or, if hydrogen is present on the nitrogen atom, the carbonyl may be converted to, e.g., chloride, with removal of HCl, followed by reaction with hydrogen sulfide. Carbamoyl chlorides are available in the art or they may be prepared from the desired amine and phosgene or thiophosgene for use in making compounds containing the

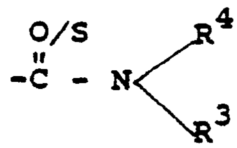

group.

The reaction of an amine with a sulfonyl chloride, e.g., $R^5NH_2 + R^6SO_2Cl$ provides the group

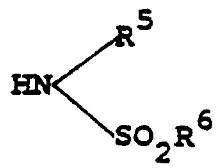

for use in reacting with an appropriate acid chloride.

The reaction of an amine with BrCN provides, e.g.,

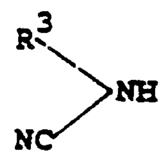

which reacts with the appropriate acid chloride to provide compounds containing the

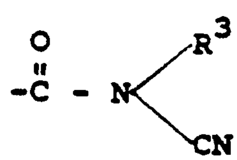

moiety. $P_2S_5$ is employed to make the corresponding S-containing compound.

Reaction of the above cyanoamine with phosgene or thiophosgene provides

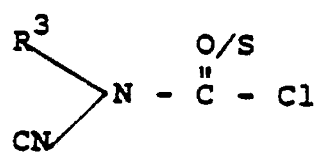

for use in making the corresponding derivatives.

The reaction of the compounds having the moiety

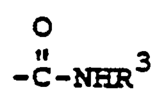

with $PCl_5$ will provide compounds having the moiety

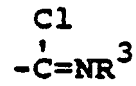

The reaction of the corresponding acid chloride with $RONH_2$ will provide compounds having the group

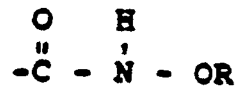

Various hydrazine derivatives can be made, e.g., from trimethyl hydrazine by reaction with the acid chlorides. The reaction of the amides, e.g.,

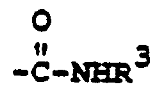

with dicarboxylic anhydrides will provide compounds having the group

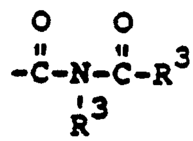

$R^2$ is preferably a carboxylic acid group, an alkali or alkaline earth metal salt thereof, an ammonium or organic amine salt thereof or a lower alkyl ester thereof, wherein "lower alkyl" includes straight, branched or cyclic saturated or unsaturated alkyl groups containing no more than 6 carbon atoms. Preferably, n is 0.

In the above formulae the aliphatic groups preferably contain 1 to 6 carbon atoms, the alkenyl and alkynyl groups preferably contain 2 to 6 carbon atoms, the alicyclic groups preferably contain 3 to 6 carbon atoms and the aromatic moiety is preferably phenyl, although other ring systems, including heterocyclic ring systems, may be employed if desired.

In the formula for the aforementioned novel compounds, X is advantageously Cl or F, and Y is advantageously $CF_3$. The group

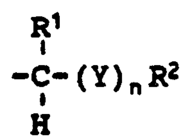

is preferably

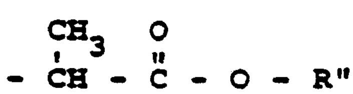

wherein R″ is methyl, ethyl, propyl, isopropyl, isobutyl or n-butyl.

The compounds of the above formula, hereinafter referred to for convenience as "active ingredients", have been found to be especially active as herbicides for the control of undesired vegetation, for example, grassy or graminaceous weeds and are unexpectedly more effective than the compounds of the known art. With the compounds of this invention, it is possible to employ lower dosage rates and still obtain effective control, thus reducing plant residues and any potential environmental contamination and/or toxicological effect on fish and warm blooded animals. Accordingly, the present invention also encompasses herbicidal compositions containing one or more of these active ingredients as well as preemergent and postemergent methods of controlling undesired plant growth, especially in the presence of valuable crops. Such methods

comprise applying a herbicidally-effective amount of one or more of said active ingredients to the locus of the undesired plants, that is, the seeds, foliage, rhizomes, stems and roots or other parts of the growing plants or soil in which the plants are growing or may be found.

The term "herbicide" is used herein to mean an active ingredient which controls or adversely modifies the growth of plants because of phytotoxic or other effects substantial enough to seriously retard the growth of the plant or further to damage the plant sufficiently to kill the plant.

By "growth controlling" or "herbicidally-effective" amount is meant an amount of active ingredient which causes a modifying effect and includes deviations from natural development, killing, regulation, desiccation, retardation, and the like.

The term "plants" is meant to include germinant seeds, emerging seedlings, rhizomes, stolons and other underground propagules, and established vegetation.

The active ingredients, i.e., new compounds, of the present invention are readily prepared by processes described in the above cited prior art and as illustrated in the following examples by choosing the appropriate starting materials. The stereoisomers are readily separated as described in European Patent 2800 referred to above.

Certain of the reactants employed to make the novel compounds of this invention are themselves novel compounds and such reactants may be made as generally described hereafter and as specifically set forth in the following examples or by methods analagous thereto, starting with known compounds.

## Example 1 - Preparation of Methyl 2-(2-fluoro-4-(4-bromo-2-fluorophenoxy)phenoxy)propionate

### A. Methyl 2-(2-fluoro-4-(2-fluoro-4-nitrophenoxy)phenoxy)propionate

To a stirred mixture of methyl 2-(2-fluoro-4-hydroxyphenoxy)propionate (4.0 g, 0.019 mole), potassium carbonate (2.9 g, 0.021 mole) and dimethyl sulfoxide (25 ml), was added 3,4-difluoronitrobenzene (3.0 g, 0.019 mole), all at once. The temperature was raised to 50 °C, and held there for 19 hours. After this time, the resultant mixture was cooled to room temperatue, then diethyl ether (50 ml) was added. This was filtered, and the filtrate was diluted with additional diethyl ether (150 ml). The latter solution was washed with 2N HCl (2 × 100 ml), then water (3 × 100 ml). After drying ($MgSO_4$) and filtering, the diethyl ether was removed by distillation. A brown viscous oil resulted weighing 6.2 grams (92 percent). NMR spectroscopy ($^1H$, $^{19}F$) confirmed the structure as that consistent with the assigned structure for the desired product. It was used directly in the next step without further purification.

### B. Methyl 2-(4-(4-amino-2-fluorophenoxy)-2-fluorophenoxy)propionate

A mixture of methyl 2-(2-fluoro-4-(2-fluoro-4-nitrophenoxy)phenoxy)propionate (6.5 g, 0.018 mole) in ethanol (150 ml) containing 5 percent palladium on charcoal (0.5 g) as catalyst was subjected to hydrogenation (initial pressure equals 50 psi) on a Paar apparatus. After 19 hours, the excess hydrogen was removed and nitrogen was bubbled through the liquid mixture. This mixture was filtered through celite and the ethanol removed by distillation. A slightly yellow oil weighing 5.8 grams (98 percent) resulted. NMR spectroscopy ($^1H$, $^{19}F$) and infrared (I.R.) spectroscopy confirmed the structure as one being consistent with the desired product. It was used directly in the next step without further purification.

### C. Methyl 2-(2-fluoro-4-(4-bromo-2-fluorophenoxy)phenoxy)propionate

To a stirred mixture of methyl 2-(4-(4-amino-2-fluorophenoxy)-2-fluorophenoxy)propionate (5.7 g, 0.018 mole), cuprous bromide (2.9 g, 0.01 mole), and hydrobromic acid (48 percent, 30 ml) cooled to less than 10 °C, was added a solution of sodium nitrite (1.5 g, 0.022 mole) in 10 ml of water. Stirring was continued for 15 minutes at 8-10 °C. The temperature was raised after the latter time to 60 °C, and held there for one half hour. The resultant mixture was cooled to room temperature, then extracted with ether (3 × 50 ml). This ether extract was washed with water (3 × 50 ml), dried ($MgSO_4$), filtered, and the ether removed by distillation. The resultant brown oil was diluted with methanol (50 ml), to which sulfuric acid (0.2 gram) was added. This solution was heated at reflux for one and one half hours. The methanol was evaporated and the

resultant red-brown oil was dissolved in ether (50 ml). This was washed with water (3 × 50 ml), treated with charcoal, then filtered through a short pad of silica gel. Evaporation of the ether gadve 1.8 g (26 percent) of a viscous amber oil: Refractice index = 1.5472 @ 25 °C. NMR spectroscopy ($^1H$, $^{19}F$) confirmed that the structure was consistent with the desired product. The carbon and hydrogen content was as follows:

| Analysis: | %C | %H |
|---|---|---|
| Calculated | 49.63 | 3.43 |
| Found: | 50.19 | 3.36 |

Example 2 - Preparation of Methyl 2-(2-fluoro-4-(2-fluoro-4-trifluoromethylphenoxy)phenoxy)propionate

A stirred solution of methyl 2-(2-fluoro-4-hydroxyphenoxy)propionate (2.0 g, 9.34 mole), 2,3-difluoro-5-trifluoromethylpyridine (1.71 g, 9.34 mole), and potassium carbonate (2.13 g, 15.3 mmole) in dimethyl sulfoxide (40 ml) was heated at 70 °C for 20 hours. After cooling, diethyl ether (100 ml) was added and the resultant mixture was stirred for five minutes. It then was filtered. The filtrate was washed sequentially with 2N HCl (2 × 100 ml) then water (3 × 100 ml). The separated ether phase was then dried ($MgSO_4$), filtered and the ether removed by distillation. A yellow oil weighing 2.8 grams (79 percent) resulted. R.I. = 1.4932 @ 25 °C. NMR spectroscopy ($^1H$, $^{19}F$) confirmed the structure as that consistent with the assigned structure for methyl 2-(2-fluoro-4-((3-fluoro-5-trifluoromethylpyridinyl)-2-oxy)-phenoxy)propionate. The carbon, hydrogen and nitrogen content was as follows:

| Analysis: | %C | %H | %N |
|---|---|---|---|
| Calculated | 50.93 | 3.21 | 3.71 |
| Found: | 50.78 | 3.26 | 3.89 |

In a similar manner, the following derivative was prepared:

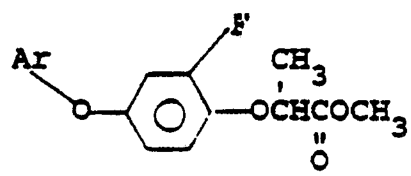

| Ar | RI or MP | Analysis Calc'd | Analysis Found |
|---|---|---|---|
| 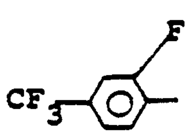 | 1.4923 | C 54.26<br>H 3.49 | C 54.46<br>H 3.47 |

The compounds of the present invention have been found to be suitable for use in methods for the selective pre- and postemergent control of annual and perennial grassy weeds. These compounds, the active ingredients of the present invention, have been found to have advantage over prior art compounds in the control of annual and perennial grassy weeds in that the present compounds control such weeds at substantially lower dosage rates. In addition, the present compounds are sufficiently tolerant towards most broad leafed crops to contemplate control of grassy weeds therein at substantially commercially practicable levels, particularly so with the preferred compounds. In addition, certain of the compounds have sufficient

tolerance towards cereal crops such as wheat to enable selective grassy weed control in these crops as well.

For such uses, unmodified active ingredients of the present invention can be employed. However, the present invention embraces the use of the compounds in composition form with an inert material, known in the art as an agricultural adjuvant or carrier, in solid or liquid form. Thus, for example, an active ingredient can be dispersed on a finely-divided solid and employed therein as a dust or granule. Also, the active ingredients, as liquid concentrates or solid compositions comprising one or more of the active ingredients can be dispersed in water, typically with aid of a wetting agent, and the resulting aqueous dispersion employed as a spray. In other procedures, the active ingredients can be employed as a constituent of organic liquid compositions, oil-in-water and water-in-oil emulsions or water dispersions, with or without the addition of wetting, dispersing, or emulsifying agents. Suitable adjuvants of the foregoing type are well known to those skilled in the art.

The herbicidally effective concentration of the active ingredients in solid or liquid compositions generally is from 0.0003 to 95 percent by weight or more. Concentrations from 0.05 to 50 percent by weight are often employed. In compositions to be employed as concentrates, the active ingredient can be present in a concentration from 5 to 98 weight percent. The active ingredient compositions can also contain other compatible additaments, for example, phytotoxicants, plant growth regulants and other biologically active compounds used in agriculture.

In further embodiments, the compounds of the present invention or compositions containing the same, can be advantageously employed in combination with one or more additional pesticidal compounds. Such additional pesticidal compounds may be insecticides, nematocides, miticides, arthropodicides, herbicides, fungicides or bactericides that are compatible with the compounds of the present invention in the medium selected for application and not antagonistic to the activity of the present compounds. Accordingly, in such embodiments, the pesticidal compound is employed as a supplemental toxicant for the same or for a different pesticidal use or as an additament. The compounds in combination can generally be present in a ratio of from 1 to 100 parts of the compound of the present invention with from 100 to 1 parts of the additional compound(s).

The active ingredients of the present invention have been found to possess desirable herbicidal activity in general against grassy weeds such as foxtail, barnyardgrass, wild oats, seedling johnsongrass and crabgrass in preemergent operations and also against the same grasses in postemergent operations while being tolerant to important broadleaf crops such as cotton, soybeans, sugarbeets and rape in the case of certain of the compounds, certain cereal crops such as wheat. These compounds are also uniquely effective in selectively controlling perennial grassy weeds as johnsongrass, quackgrass, bermudagrass and dallisgrass.

The active ingredients of the present invention have been found to possess particularly desirable herbicidal activity against wild oats, foxtail, barnyardgrass, crabgrass and seedling johnsongrass in postemergent operations as well as desirable broad spectrum activity against the perennial grassy weeds listed above and at lower dosage rates than the substituted propanoates and propanols of the prior art while showing high selectivity to broadleaf crops and, in the case of certain of the compounds, wheat.

The exact rate to be applied is dependent not only on a specific active ingredient being applied, but also on a particular action desired, the plant species to be modified and the stage of growth thereof as well as the part of the plant to be contacted with the toxic active ingredient. Thus, all of the active ingredients of the present invention and compositions containing the same may not be equally effective at similar concentrations or against the same plant species.

In postemergent operations a dosage of 0.005 to 20 pounds/acre (0.0056 -22.4 kg/hectare) is generally applicable, although not all compounds are equally effective and some weeds are more difficult to control. Thus, a dosage rate in the range of 0.01 to 1.0 pound/acre (0.01-1.12 kg/hectare) is preferred in postemergent control of annual grassy weeds, while 0.05 to 5 pounds/acre (0.056-5.6 kg/hectare) is a preferred dosage range for the postemergent control of perennial grassy weeds. In applications to tolerant crops a weed controlling but less than crop damaging amount of from .005 to 1.0 lb/acre (0.0056 to 1.12 kgs/hectare) is generally employed.

In preemergent operations a dosage rate of 0.01 to 10 lbs/acre (0.011 to 11.2 kgs/hectare), preferably 0.05 to 2.0 lbs/acre (0.056 to 2.25 kgs/hectare) and most preferably 0.1 to 1 lb/acre (0.11 to 1.12 kgs/hectare) is generally employed.

The following examples illustrate effects of the compounds of this invention.

Example 3

In representative operations, each compound to be utilized in a series of tests is dissolved in acetone to one-half of the final volume (twice the final concentration) to be used and the acetone solution in each case is admixed with an equal volume of water containing 0.1 percent by weight of the non-ionic surfactant TWEEN® 20 (a polyoxyethylene sorbitan monolaurate). The compositions, generally in the nature of an emulsion, were employed to spray separate respective plant species which had been grown to a height of 2-6 inches in soil of good nutrient content in a greenhouse. Sufficient amounts were employed to provide various application rates as listed in the table. The various beds were positioned side by side and exposed to substantially identical conditions of temperature and light. Each bed was maintained so as to prevent any interaction with test compounds in different seed beds. Other portions of the plants were left untreated to serve as controls. After treatment, the plants were maintained for about two weeks under greenhouse conditions conducive for good plant growth and watered as necessary. The specific plant species, test compound and dosage and the percent postemergent control obtained are set forth in the table below. Control refers to the reduction in growth compared to the observed results of the same untreated species. Note the "NT" means "not tested".

Plant species in these tests were the following:

| Common Name | Scientific Name |
|---|---|
| Barnyardgrass (Watergrass) | *Echinochloa crusgalli* |
| Crabgrass | *Digitaria sanguinalis* |
| Yellow foxtail | *Setaria lutescens* |
| Johnson grass | *Sorghum halepense* |
| Wild Oats | *Avena fatua* |

POSTEMERGENT CONTROL OF PLANT SPECIES

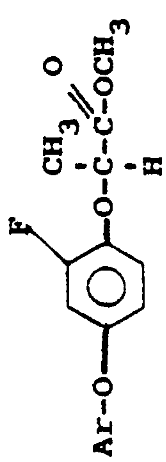

| Ar | Plant Species | Percent Control at Indicated Dosage (ppm) 250 | 125 | 62.5 | 31.25 | 15.6 | 7.8 |
|---|---|---|---|---|---|---|---|
| 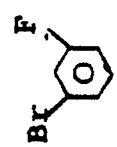 | Barnyardgrass | 100 | 100 | 100 | 100 | 0 | 0 |
| | Crabgrass | 100 | 100 | 100 | 100 | 95 | 0 |
| | Foxtail | 100 | 100 | 90 | 75 | 75 | 0 |
| | Johnsongrass | 35 | 20 | 10 | 0 | 0 | 0 |
| | Wild Oats | 40 | 20 | 0 | 0 | 0 | 0 |

Example 7

So as to clearly illustrate the phytotoxic properties of the various active ingredients of the present invention applied preemergently, a controlled greenhouse experiment is described below.

The seeds of various species of plants were planted in beds of good agricultural soil in a greenhouse. A number of compositions of the present invention, generally in the nature of an aqueous emulsion, were applied at rates listed in the table so as to deposit a predetermined amount of active ingredients uniformly throughout the surface of the bed. Another seed bed was treated only with water to serve as a control. After

treatment, the seed beds were maintained for two weeks under greenhouse conditions conductive for good plant growth and watered as necessary. The specific plant species, test compound, and dosage and the percent preemergent control are set forth in the table below. Control refers to the reduction in growth compared to the observed results of the same untreated species.

PREEMERGENT CONTROL OF PLANT SPECIES

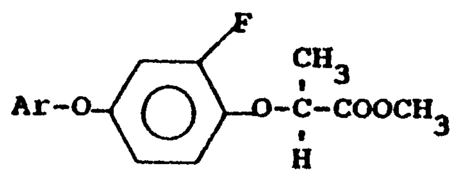

| Ar | Plant Species | Dosage (lb/acre, kg/hectare) 0.5 (0.56) | 0.25 (0.28) | 0.125 (0.14) | 0.063 (0.07) | 0.031 (0.035) |
|---|---|---|---|---|---|---|
| 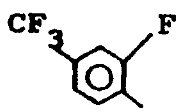 | Cotton | NT | 0 | 0 | 0 | 0 |
| | Rape | NT | 0 | 0 | 0 | 0 |
| | Soybean | NT | 0 | 0 | 0 | 0 |
| | Sugarbeet | NT | 0 | 0 | 0 | 0 |
| | Rice | NT | 100 | 90 | 70 | 20 |
| | Wheat | NT | 70 | 0 | 0 | 0 |
| | Barnyardgrass | NT | 100 | 75 | 30 | 0 |
| | Johnsongrass | NT | 100 | 100 | 25 | 0 |
| | Wild Oats | NT | 85 | 80 | 25 | 0 |
| | Yellow Foxtail | NT | 100 | 100 | 20 | 0 |

PREEMERGENT CONTROL OF PLANT SPECIES (Continued)

| Ar | Plant Species | Dosage (lb/acre, kg/hectare) 0.5 (0.56) | 0.25 (0.28) | 0.125 (0.14) | 0.063 (0.07) | 0.031 (0.035) |
|---|---|---|---|---|---|---|
| 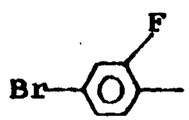 | Cotton | 0 | 0 | 0 | 0 | 0 |
| | Rape | 0 | 0 | 0 | 0 | 0 |
| | Soybean | 0 | 0 | 0 | 0 | 0 |
| | Sugarbeet | 0 | 0 | 0 | 0 | 0 |
| | Rice | 70 | 0 | 0 | 0 | 0 |
| | Wheat | 0 | 0 | 0 | 0 | 0 |
| | Barnyardgrass | 100 | 100 | 95 | 30 | 0 |
| | Johnsongrass | 99 | 40 | 0 | 0 | 0 |
| | Wild Oats | 90 | 30 | 10 | 0 | 0 |
| | Yellow Foxtail | 100 | 100 | 50 | 0 | 0 |

## Claims

1. A compound having the formula

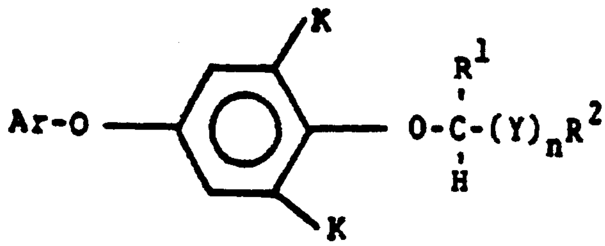

wherein Ar is a substituted or unsubstituted phenyl group;
K is H or F with the proviso that at least one K is F;
Y is a saturated or unsaturated alkyl groups containing an even number of carbon atoms;
n is 0 or 1;
$R^1$ is H or a $C_1$-$C_3$ alkyl group;
$R^2$ is a carboxyl group; an alkali metal, alkaline earth metal, ammonium or organic amine salt thereof; or an organic group containing N, O or S atoms that can be hydrolyzed and/or oxidised in plants or the soil to a carboxyl group that is in undissociated and/or dissociated form.

2. A compound as claimed in claim 1 wherein Ar is the group

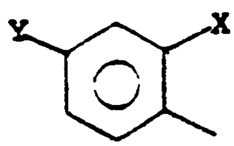

wherein X is hydrogen or halogen and Y is halogen, $CF_3$, $CHF_2$ or $CClF_2$.

3. A compound as claimed in claim 1 or claim 2 wherein $R^1$ is a methyl group; n is O; and $R^2$ is the group COOR″ wherein R″ is hydrogen, methyl, ethyl, propyl, isopropyl, isobutyl or n-butyl.

4. A compound as claimed in claim 3 wherein R″ is hydrogen.

5. A compound as claimed in claim 3 wherein R″ is methyl.

6. A compound as claimed in claim 1 of the formula

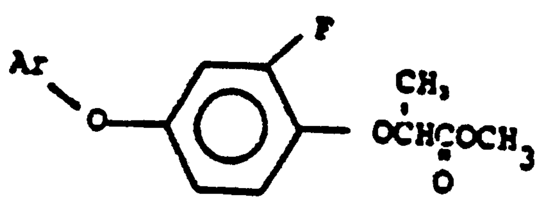

wherein the group Ar is

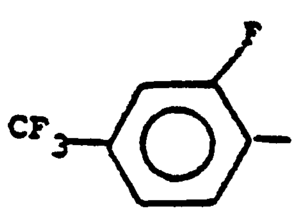

7. The R-enantiomer of a compound as claimed in any one of the preceding claims.

8. Methyl 2-(2-fluoro-4-(4-bromo-2-fluorophenoxy)phenoxy)propionate.

9. A herbicidal composition comprising an inert adjuvant or carrier and herbicidally effective amount of a compound having the formula

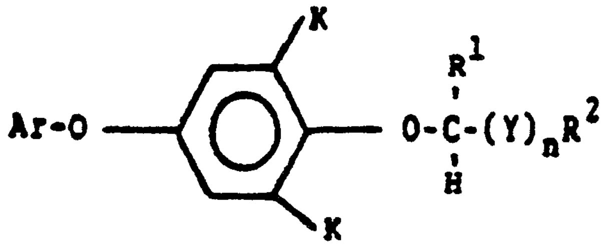

wherein Ar, K, Y, $R^1$, $R^2$ and n are as defined in claim 1 or an R-enantiomer thereof.

10. A composition as claimed in claim 9 wherein the said compound is a compound as defined in any one of claims 2 to 8.

11. A method of controlling undesired plant growth which comprises applying to the locus of the plants a composition as claimed in claim 9 or claim 10.

12. A method as claimed in claim 11 wherein the composition is applied postemergently to grassy weeds.

13. A method as claimed in claim 12 wherein the composition is applied at a dosage rate sufficient to provide from 0.005 to 20 pounds/acre (0.0056 - 22.4kg/hectare) of the active ingredient.

14. A method as claimed in claim 11 wherein the composition is applied pre-emergently.

15. A method as claimed in claim 14 wherein the composition is applied pre-emergently at a dosage rate sufficient to provide from 0.05 to 2.0lbs/acre (0.056 to 2.25 kgs/hectare) of the active ingredient.

16. A method of killing or controlling vegetation, which method comprises providing in the vegetation a compound of the formula

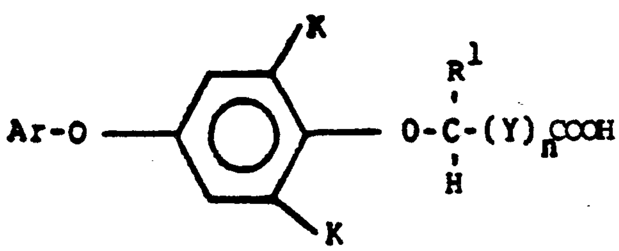

where Ar, K, $R^1$, Y and n are as defined in claim 1.

Claims for the following contracting State: AT

1. A herbicidal composition comprising an inert adjuvant or carrier and a herbicidally effective amount of a compound having the formula

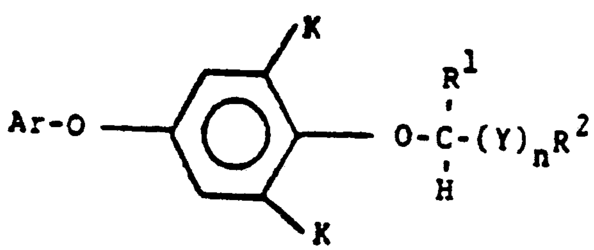

wherein Ar is a substituted or unsubstituted phenyl group;
K is H or F with the proviso that at least one K is F;
Y is a saturated or unsaturated alkyl group containing an even number of carbon atoms;

n is 0 or 1;
$R^1$ is H or a $C_1$-$C_3$ alkyl group;
$R^2$ is a carboxyl group, an alkali metal, alkaline earth metal, ammonium or organic amine salt thereof; or an organic group containing N, O or S atoms that can be hydrolyzed and/or oxidised in plants or the soil to a carboxyl group that is in undissociated and/or dissociated form, or an R-enantiomer thereof.

2. A composition as claimed in claim 1 wherein Ar is the group

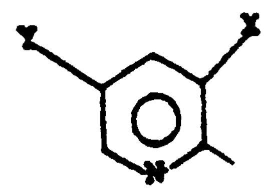

wherein X is hydrogen or halogen and Y is halogen, $CF_3$, $CHF_2$ or $CClF_2$.

3. A compound as claimed in claim 1 or claim 2 wherein $R^1$ is a methyl group; n is O; and $R^2$ is the group COOR″ wherein R″ is hydrogen, methyl, ethyl, propyl, isopropyl, isobutyl or n-butyl.

4. A composition as claimed in claim 3 wherein R″ is hydrogen.

5. A composition as claimed in claim 3 wherein R″ is methyl.

6. A composition as claimed in claim 1 wherein the compound has the formula

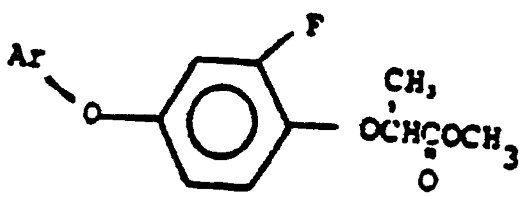

wherein the group Ar is

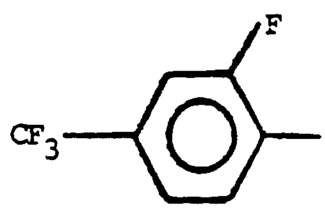

7. A composition as claimed in claim 1 wherein the compound is methyl-2-(2-fluoro-4-(4-bromo-2-fluorophenoxy)phenoxy)propionate.

8. A method of controlling undesired plant growth which comprises applying to the locus of the plants a composition as claimed in any one of claims 1 to 5.

9. A method as claimed in claim 8 wherein the composition is applied postemergently to grassy weeds.

10. A method as claimed in claim 9 wherein the composition is applied at a dosage rate sufficient to provide from 0.005 to 20 pounds/acre (0.0056 -22.4kg/hectare) of the active ingredient.

11. A method as claimed in claim 8 wherein the composition is applied preemergently.

12. A method as claimed in claim 11 wherein the composition is applied preemergently at a dosage rate sufficient to provide from 0.05 to 2.0lbs/acre (0.056 to 2.25kgs/hectare) of the active ingredient.

13. A method of killing or controlling vegetation, which method comprises providing in the vegetation a compound of the formula

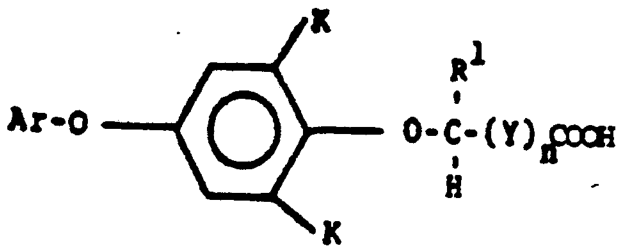

where Ar, K, $R^1$, Y and n are as defined in claim 1.